# EUROPEAN PATENT APPLICATION

(11) **EP 2 636 726 A1**
(43) Date of publication of application: **11.09.2013**
(21) Application number: 11838101.1
(22) Date of filing: 04.11.2011
(51) Int. Cl.: C11B 5/00, A23K 1/00, A23K 1/16, C07C 51/50, C07C 65/05

(54) **CASHEW NUTSHELL OIL OF IMPROVED STABILITY**

(30) Priority: 05.11.2010 JP 2010249188
(71) Applicant: Idemitsu Kosan Co., Ltd., Chiyoda-ku Tokyo 100-8321 (JP)
(72) Inventor: OOIWA, Seika, Sodegaura-shi, Chiba 299-0293 (JP); NAGASHIMA, Kyo, Sodegaura-shi, Chiba 299-0293 (JP); ITO, Shinji, Sodegaura-shi, Chiba 299-0293 (JP); MOCHIZUKI, Masami, Sodegaura-shi, Chiba 299-0293 (JP)
(74) Representative: Vossius & Partner
(86) International application number: PCT/JP2011/075496
(87) International publication number: WO 2012/060451

(57) **Abstract**

The present invention provides a composition comprising a chelating agent and unheated cashew nut shell liquid.

## Description

### TECHNICAL FIELD

The present invention relates to a composition comprising a chelating agent and unheated cashew nut shell liquid (CNSL), and an unheated cashew nut shell liquid formulation and a feed which use the composition. The present invention also relates to a method for inhibiting decarboxylation reaction of anacardic acid in unheated cashew nut shell liquid, comprising adding a chelating agent to the unheated cashew nut shell liquid.

### BACKGROUND ART

Cashew nut shell liquid is oily liquid contained in the shell of the seed of a cashew nut obtained from the cashew nut tree *(Anacardium occidentale L.).* The cashew nut shell liquid mainly contains, as the components thereof, anacardic acid, cardanol, cardol and methylcardol.
A method for preparing the cashew nut shell liquid comprises a heating process and a solvent extraction process. Usually, the cashew nut shell liquid is heat-treated by the producer of cashew nuts to convert the anacardic acid into cardanol for use.
This is because unheated cashew nut shell liquid is likely to cause decarboxylation of the anacardic acid of its component at room temperature and thus can form a foam during transportation. The unheated cashew nut shell liquid is solidified and loses the liquidity at about 20°C and thus not readily transportable. For these reasons, the unheated cashew nut shell liquid has a great restriction on transportation, thereby hindering the further industrial use.

The Patent Documents 1-3 describe the industrial product uses. All of these patents use a heat-treated cashew nut shell liquid.
The Patent Documents 4-6 describe use of unheated cashew nut shell liquid and the anacardic acid which is a component of the cashew nut shell liquid, for a feed application. Given the restrictions on the stability and transportation as described above, many challenges exist including physical and economic challenges. Although the Patent Documents 4-6 describe use of unheated cashew nut shell liquid, these patents do not attempt to improve the stability and the handleability.
Thus there has been a demand for a method for stabilizing unheated cashew nut shell liquid to allow it to be readily transportable.

The Patent Document 7 describes a method for stabilizing anacardic acid by admixing alkali with cashew nut shell liquid, which contains anacardic acid. The patent, however, does not describe addition of a chelating agent such as an organic acid chelating agent and a phosphoric acid chelating agent to cashew nut shell liquid to stabilize anacardic acid. The patent does not describe addition of an inorganic carrier to the unheated cashew nut shell liquid which comprises a chelating agent to prevent the solidification at about 20°C.

### PRIOR ART DOCUMENTS

### PATENT DOCUMENTS

Patent Document 1: JP 2008-144171 A
Patent Document 2: JP 2006-111839 A
Patent Document 3: JP 2003-252893 A
Patent Document 4: JP 2003-238400 A
Patent Document 5: JP 2001-151675 A
Patent Document 6: JP 8-231410 A
Patent Document 7: JP 2010-88363 A

### SUMMARY OF THE INVENTION

The present invention is directed to make unheated cashew nut shell liquid, especially anacardic acid, which is a component of the cashew nut shell liquid, readily transportable without decarboxylation. The present invention is also directed to make unheated cashew nut shell liquid, which is solidified at about 20°C, readily transportable even at low temperature.

Through their extensive research to solve the problems described above, the present inventors have found that addition of a chelating agent to unheated cashew nut shell liquid allows inhibition of decarboxylation of the anacardic acid.
The present inventors have also found that addition of a chelating agent and then an inorganic carrier such as silica to unheated cashew nut shell liquid allows the unheated cashew nut shell liquid to be portable without solidification even at a temperature equal to or lower than about 20°C.
In this way, the present inventors have completed the present invention.

The present invention relates to
(1) a composition comprising a chelating agent and unheated cashew nut shell liquid;
(2) the composition according to (1), wherein the chelating agent is an organic acid chelating agent, an organic acid salt chelating agent, a phosphoric acid chelating agent, a phosphate chelating agent, an amino polycarboxylic acid chelating agent, an amino polycarboxylate chelating agent, a phosphonic acid chelating agent, a phosphonate chelating agent, a neutral amino acid chelating agent, an aluminosilicate chelating agent, or a polymer chelating agent;
(3) the composition according to (2), wherein the organic acid chelating agent and the organic acid salt chelating agent are citric acid or a salt thereof, malic acid or a salt thereof, tartaric acid or a salt thereof, succinic acid or a salt thereof, propionic acid or a salt thereof, gluconic acid or a salt thereof, oxalic acid or a salt thereof, or glycolic acid or a salt thereof;
(4) the composition according to (3), wherein the chelating agent is citric acid, malic acid, or tartaric acid;
(5) the composition according to (2), wherein the phosphoric acid chelating agent and the phosphate chelating agent are orthophosphoric acid or a salt thereof, pyrophosphoric acid or a salt thereof, tripolyphosphoric acid or a salt thereof, tetrapolyphosphoric acid or a salt thereof, hexametaphosphoric acid or a salt thereof, or phytic acid or a salt thereof;
(6) the composition according to (5), wherein the chelating agent is orthophosphoric acid or a salt thereof;
(7) the composition according to (2), wherein the amino polycarboxylic acid chelating agent and the amino polycarboxylate chelating agent are ethylenediamine tetraacetic acid or a salt thereof, ethylenediamine diacetic acid or a salt thereof, hydroxyethyl ethylenediamine tetraacetic acid or a salt thereof, diethylenetriamine pentaacetic acid or a salt thereof, nitrilotriacetic acid or a salt thereof, triethylenetetraamine hexaacetic acid or a salt thereof, dicarboxymethyl glutamine hexaacetic acid or a salt thereof, dicarboxymethyl glutamic acid tetrasodium salt, or dihydroxymethyl glycine;
(8) the composition according to (7), wherein the chelating agent is ethylenediamine tetraacetic acid;
(9) the composition according to (2), wherein the neutral amino acid chelating agent is glycine, alanine, leucine, cysteine, methionine, asparagine, or glutamine;
(10) the composition according to (9), wherein the chelating agent is glycine;
(11) the composition according to (2), wherein the aluminosilicate chelating agent is zeolite;
(12) the composition according to (2), wherein the polymer chelating agent is polyacrylic acid, polymaleic acid, or a copolymer of acrylic acid and maleic acid;
(13) the composition according to any one of (1) - (12), wherein an amount of the chelating agent is equal to or greater than 0.05% by weight based on the weight of the cashew nut shell liquid;
(14) an unheated cashew nut shell liquid formulation comprising the composition according to any one of (1)-(13) and an inorganic carrier;
(15) the unheated cashew nut shell liquid formulation according to (14), wherein the inorganic carrier is silica;
(16) a feed containing unheated cashew nut shell liquid, comprising the composition according to any one of (1)-(13) and/or the unheated cashew nut shell liquid formulation according to (14) or (15);
(17) a method for inhibiting decarboxylation reaction of the anacardic acid in unheated cashew nut shell liquid, comprising adding a chelating agent to the unheated cashew nut shell liquid;
(18) a method for producing the unheated cashew nut shell liquid formulation according to (14) or (15), comprising mixing a chelating agent with an inorganic carrier and then mixing the mixture of the chelating agent and the inorganic carrier with unheated cashew nut shell liquid; and
(19) a method for inhibiting decarboxylation reaction of anacardic acid, comprising adding a chelating agent to the anacardic acid.

### EFFECTS OF THE INVENTION

Addition of a chelating agent to unheated cashew nut shell liquid allows inhibition of decarboxylation of the anacardic acid which is a component of the unheated cashew nut shell liquid. Addition of an inorganic carrier to the mixture of the unheated cashew nut shell liquid and the chelating agent allows inhibition of solidification of the unheated cashew nut shell liquid at about 20°C. These facilitate industrial use of unheated cashew nut shell liquid.

### BRIEF DESCRIPTION OF THE DRAWINGS

FIG. 1 illustrates inhibition of decarboxylation reaction in unheated cashew nut shell liquid when 2 or 10% by weight of citric acid, 2 or 10% by weight of malic acid, or 2 or 10% by weight of tartaric acid was used. The bar graphs, as well as the graphs in the figures that follow, illustrate cardanol 15:1, cardanol 15:2, cardanol 15:3, anacardic acid 15:1, anacardic acid 15:2, and anacardic acid 15:3 from top to bottom.
FIG. 2 illustrates inhibition of decarboxylation reaction in unheated cashew nut shell liquid when 10% by weight of phosphoric acid or sodium dihydrogen phosphate was used.
FIG. 3 illustrates inhibition of decarboxylation reaction in unheated cashew nut shell liquid when 1 or 5% by weight of EDTA was used.
FIG. 4 illustrates inhibition of decarboxylation reaction in unheated cashew nut shell liquid when 1 or 10% by weight of glycine was used.
FIG. 5 illustrates inhibition of decarboxylation reaction in unheated cashew nut shell liquid when 1 or 10% by weight of zeolite was used.
FIG. 6 illustrates inhibition of decarboxylation reaction in unheated cashew nut shell liquid when 2 or 10% by weight of citric acid was used.
FIG. 7 illustrates inhibition of decarboxylation reaction in an unheated cashew nut shell liquid formulation when 10% by weight of phosphoric acid or sodium dihydrogen phosphate was used.
FIG. 8 illustrates inhibition of decarboxylation reaction in a feed when 10% by weight of phosphoric acid or citric acid was used.

### MODE FOR CARRYING OUT THE INVENTION

The composition of the present invention comprises a chelating agent and unheated cashew nut shell liquid.

The cashew nut shell liquid used in the present invention is oily liquid contained in the shell of the seed of a cashew nut obtained from the cashew nut tree (*Anacardium occidentale L.).* The cashew nut shell liquid contains, as the components thereof, anacardic acid, cardanol, cardol, and methylcardol.
Unheated cashew nut shell liquid extracted by compressing the shell of a cashew nut (hereinafter referred to as "cashew nut shell liquid") usually contains 55-80% by mass of anacardic acid, 5-20% by mass of cardanol, and 5-30% by mass of cardol, as described in J. Agric. Food Chem. 2001, 49, 2548-2551.
There are three types of anacardic acid: the anacardic acid which has three double bonds at 8-, 11-, and 14-positions (hereinafter referred to as "15:3"), the anacardic acid which has two double bonds at 8- and 11-positions (hereinafter referred to as "15:2"), and the anacardic acid which has a double bond at 8-position.
The present invention also includes a method for inhibiting decarboxylation reaction of anacardic acid, comprising adding a chelating agent to the anacardic acid.

The cashew nut shell liquid used in the present invention can be obtained as a vegetable oil extracted by compressing the shell of a cashew nut. The cashew nut shell liquid used in the present invention can also be obtained by extracting, for example, solvent-extracting a cashew nut shell. The cashew nut shell liquid used in the present invention can be obtained by the process described in JP 8-231410 such as, for example, solvent extraction.
The cashew nut shell liquid used in the present invention may be obtained by pulverizing/crushing the shell of a cashew nut.
The cashew nut shell liquid used in the present invention may be a commercially-available product.

In the present invention, a chelating agent refers to a multidentate ligand which forms coordinate bonds with a metal ion contained in cashew nut shell liquid to produce a chelate compound.
The chelating agent used in the present invention includes organic acid chelating agents, organic acid salt chelating agents, phosphoric acid chelating agents, phosphate chelating agents, amino polycarboxylic acid chelating agents, amino polycarboxylate chelating agents, neutral amino acid chelating agents, aluminosilicate chelating agents, phosphonic acid chelating agents, phosphonate chelating agents, and polymer chelating agents.
The organic acid chelating agents and the organic acid salt chelating agents include citric acid and salts thereof, malic acid and salts thereof, tartaric acid and salts thereof, succinic acid and salts thereof, gluconic acid and salts thereof, oxalic acid and salts thereof, glycolic acid and salts thereof, and propionic acid and salts thereof.
The phosphoric acid chelating agents and the phosphate chelating agents include orthophosphoric acid and salts thereof, pyrophosphoric acid and salts thereof, tripolyphosphoric acid and salts thereof, tetrapolyphosphoric acid and salts thereof, hexametaphosphoric acid and salts thereof, and phytic acid and salts thereof. The salts of orthophosphoric acid include sodium dihydrogen phosphate, potassium dihydrogen phosphate, ammonium dihydrogen phosphate, disodium hydrogen phosphate, dipotassium hydrogen phosphate, and diammonium hydrogen phosphate. The amino polycarboxylic acid chelating agents and the amino polycarboxylate chelating agents include ethylenediamine tetraacetic acid (EDTA) and salts thereof, ethylenediamine diacetic acid and salts thereof, hydroxyethyl ethylenediamine tetraacetic acid and salts thereof, diethylenetriamine pentaacetic acid and salts thereof, nitrilotriacetic acid and salts thereof, triethylenetetraamine hexaacetic acid and salts thereof, dicarboxymethyl glutamine hexaacetic acid and salts thereof, dicarboxymethyl glutamic acid tetrasodium salt, dihydroxymethyl glycine, 1,3-propanediamine tetraacetic acid and salts thereof, 1,3-diamino-2-hydroxypropane tetraacetic acid and salts thereof, phosphonobutane tricarboxylic acid and salts thereof, glutamic acid and salts thereof, cyclohexanediamine tetraacetic acid and salts thereof, iminodiacetic acid and salts thereof, N-(2-hydroxyethyl)iminodiacetic acid and salts thereof, N-(2-hydroxyethyl)ethylenediamine triacetic acid and salts thereof, glycol ether diaminetetraacetic acid and salts thereof, glutamic acid diacetic acid and salts thereof, aspartic acid diacetic acid and salts thereof, and dihydroxymethyl glycine.
The neutral amino acid chelating agents include glycine, alanine, leucine, cysteine, methionine, asparagine, and glutamine.
The aluminosilicate chelating agents include zeolite.
The phosphonic acid chelating agents and the phosphonate chelating agents include hydroxyethylidene diphosphonic acid and salts thereof, nitrilotris methylene phosphonic acid and salts thereof, and nitrilotris and salts thereof.
The polymer chelating agents include polyacrylic acid, polymaleic acid, and a copolymer of maleic acid and acrylic acid.

The organic acid chelating agent and the organic acid salt chelating agent are used preferably in an amount equal to or greater than 0.05% by weight, more preferably in an amount of 0.1-15% by weight, and still more preferably in an amount of 0.5-10% by weight based on the weight of the unheated cashew nut shell liquid (CNSL).
The phosphoric acid chelating agent and the phosphate chelating agent are used preferably in an amount equal to or greater than 0.05% by weight, more preferably in an amount of 0.1-15% by weight, and still more preferably in an amount of 0.5-10% by weight based on the weight of the unheated cashew nut shell liquid.
The amino polycarboxylic acid chelating agent and the amino polycarboxylate chelating agent are used preferably in an amount equal to or greater than 0.05% by weight, more preferably in an amount of 0.1-20% by weight, and still more preferably in an amount of 0.5-15% by weight based on the weight of the unheated cashew nut shell liquid.
The neutral amino acid chelating agent is used preferably in an amount equal to or greater than 0.1% by weight, more preferably in an amount of 0.5-20% by weight, and still more preferably in an amount of 1-20% by weight based on the weight of the unheated cashew nut shell liquid.
The aluminosilicate chelating agent is used preferably in an amount equal to or greater than 0.1% by weight, more preferably in an amount of 0.5-20% by weight, and still more preferably in an amount of 1-20% by weight based on the weight of the unheated cashew nut shell liquid.
The phosphonic acid chelating agent and the phosphonate chelating agent are used preferably in an amount equal to or greater than 0.05% by weight, more preferably in an amount of 0.1-20% by weight, and still more preferably in an amount of 0.5-15% by weight based on the weight of the unheated cashew nut shell liquid.
The polymer chelating agent is used preferably in an amount equal to or greater than 0.05% by weight, more preferably in an amount of 0.1-5% by weight, and still more preferably in an amount of 0.5-3% by weight based on the weight of the unheated cashew nut shell liquid.
The two or more chelating agents may be used in combination. The chelating agents are used preferably in a total amount equal to or less than 0.1-30% by weight, more preferably in a total amount equal to or less than 0.5-25% by weight, and still more preferably in a total amount equal to or less than 1.0-20% by weight.
The composition of the present invention can be produced by adding a chelating agent to an unheated cashew nut.

The unheated cashew nut shell liquid formulation of the present invention is preferably a formulation which comprises the composition of the present invention and an inorganic carrier. For example, the composition of the present invention can be mixed with an inorganic carrier to produce the unheated cashew nut shell liquid formulation.
The inorganic carrier includes, but not limited to, silicic acid and salts thereof (for example, silica), vermiculite, diatomaceous earth, talc, kaolin, and bentonite.
When silicic acid or a salt thereof is used as the inorganic carrier, it has preferably a specific surface area of 500 m²/g or less to prevent significant oxidation. When silica is used as the inorganic carrier, the compounding ratio (based on weight), silica/unheated cashew nut shell liquid, is preferably in the range from 1/3.0 to 1/0.1. The silica/unheated cashew nut shell liquid ratio is more preferably in the range from 1/2.5 to 1/0.5, and still more preferably in the range from 1/2.0 to 1/1.0. When another inorganic carrier is used, it may have the similar specific surface area and the similar compounding ratio. The specific surface area of silica can be measured by the BET method.
The unheated cashew nut shell liquid formulation of the present invention is not particularly limited as long as it includes unheated cashew nut shell liquid, an inorganic carrier, and a chelating agent. Thus the unheated cashew nut shell liquid formulation may be produced by premixing a chelating agent with an inorganic carrier and then mixing the premix of the chelating agent and the inorganic carrier with unheated cashew nut shell liquid. The chelating agent mixed with the inorganic carrier can inhibit decarboxylation in the unheated cashew nut shell liquid.

In addition to the unheated cashew nut shell liquid, the inorganic carrier, and the chelating agent, the unheated cashew nut shell liquid formulation of the present invention may include antioxidant. The antioxidant includes, for example, ethoxyquin, t-butylhydroxytoluene, t-butylhydroxyanisole, t-butylhydroquinone, ascorbic acid and esters thereof, vitamin E, gallic acid and esters thereof, erythorbic acid, chlorogenic acid, sulfite, thiosulfate, phosphite, and hypophosphite, although not limited thereto.

The dosage form of the unheated cashew nut shell liquid formulation of the present invention can be formulated as powders by adding an inorganic carrier such as silica. In other words, the unheated cashew nut shell liquid formulation of the present invention can be produced by mixing unheated cashew nut shell liquid, an inorganic carrier, a chelating agent, and, as required, an optional component to formulate powders. Such powder formulation of the present invention may be used as a feed without mixing other optional components.

In addition to powders, the unheated cashew nut shell liquid formulation of the present invention can be formulated as granules such as pellets. In this case, hardened oil may also be added to the unheated cashew nut shell liquid, in addition to the inorganic carrier and the chelating agent. The hardened oil includes hardened palm oil, hardened soybean oil, hardened canola oil, and the like. The hardened oil preferably has a melting point of 45-65°C. The pellets can be produced using a usual extrusion granulator.

The unheated cashew nut shell liquid formulation of the present invention may be coated. For example, the formulation, after granulation, can be coated with a coating agent selected from zein, shellac, HPMC (hydroxypropylmethylcellulose), pullulan, hemilose, glucose, lactose, trehalose, and starch. The formulation also may be coated with a sheet which includes as a component the coating agent as described above.

The present invention includes a feed which includes the composition of the present invention and/or the unheated cashew nut shell liquid formulation of the present invention. In the present invention, the chelating agent inhibits the unheated cashew nut shell liquid included in the feed from causing decarboxylation.

For the feed of the present invention, any types of feed components may be combined in any ratio with the composition of the present invention and/or the cashew nut shell liquid formulation of the present invention, as long as the feed components are conventionally given to the respective animals, including, for example, corn kernels, corn flours, milo, soybean meal, oats, wheat short, wheat middlings, alfalfa, clovers, defatted rice bran, northern ocean meal, coast meal, yeast, molasses, meat pieces, bone meal, calcium carbonate, calcium diphosphate, yellow grease, vitamins, and minerals.

The unheated cashew nut shell liquid formulation can be admixd with a feed component to produce the feed of the present invention. When the unheated cashew nut shell liquid formulation in powder or solid form is used, the formulation may be formulated into liquid or gel form to facilitate the admixing. Water, vegetable oil such as soybean oil, canola oil, and corn oil, liquid animal oil, water-soluble polymer compounds such as polyvinyl alcohol and polyvinyl pyrrolidone can be used as a liquid carrier. It is preferred to add water-soluble polysaccharide such as xanthan gum, sodium caseinate, arabic gum, guar gum, and tamarind seed polysaccharide to maintain the uniformity of the cashew nut shell liquid in the feed.

The feed of the present invention is suitable for livestock such as cattle, pigs, chickens, sheep, horses, and goats. The amount of the feed supplied can be adjusted as desired, depending on the type, weight, age, sex, and health condition of the animal, the components of the feed, and the like. The cashew nut shell liquid contained in the feed is ingested preferably in an amount of 0.005-500g per animal per day and more preferably in an amount of 0.05-100g per animal per day.
Any of the usual method for feeding and raising an animal can be used depending on the type of the animal.

### EXAMPLES

### Example 1: Preparation of Samples

Cashew nut shells were obtained from Cashew Trading Co., Ltd. and compressed as necessary to produce cashew nut shell liquid (unheated cashew nut shell liquid).
Sipernat 22 silica (Evonik Degussa Japan) was used as the carrier.

### Example 2: HPLC Measurement

An HPLC system (Waters 600, Nihon Waters K.K.), a detector (Waters 490E, Nihon Waters K.K.), a printer (Chromatopac C-R6A, Shimadzu Corp.), and a column (Supelcosil LC18, Supelco, Inc.) were used. The analysis was carried out under the conditions of a solvent composition of acetonitrile: water: acetic acid = 80:20:1 (based on volume), a flow rate of 2 ml/min, a temperature of 25°C, and an absorbance of 280 nm.

### Example 3. Inhibition of Decarboxylation Reaction When Organic Acid Chelating Agent was Added

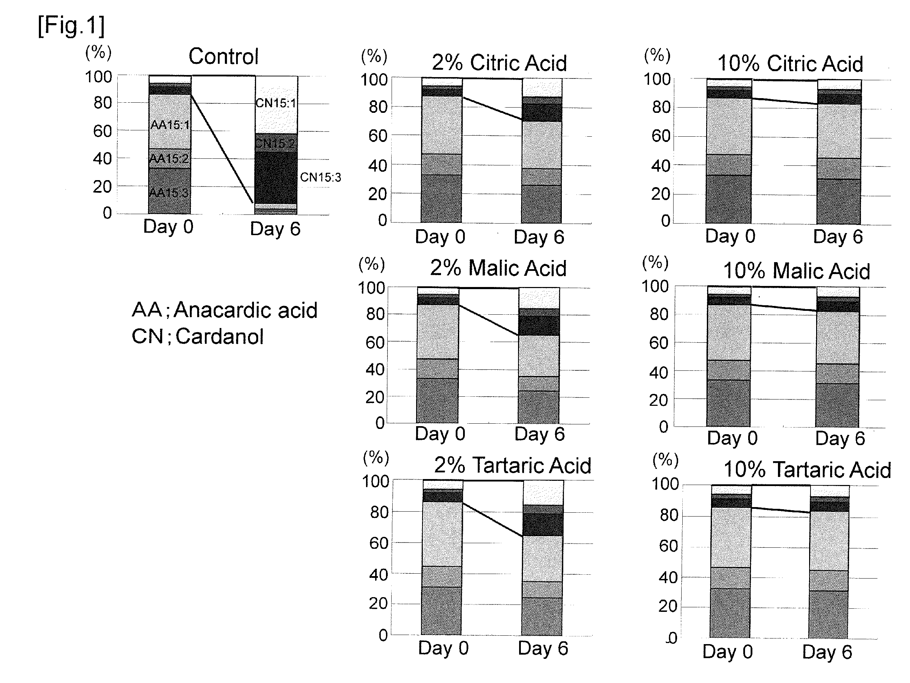

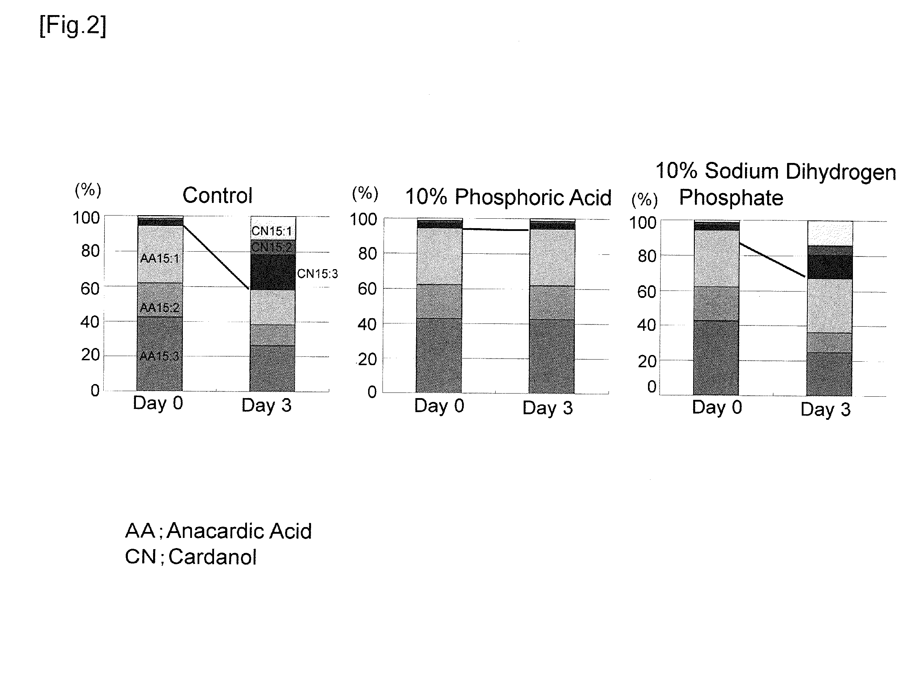

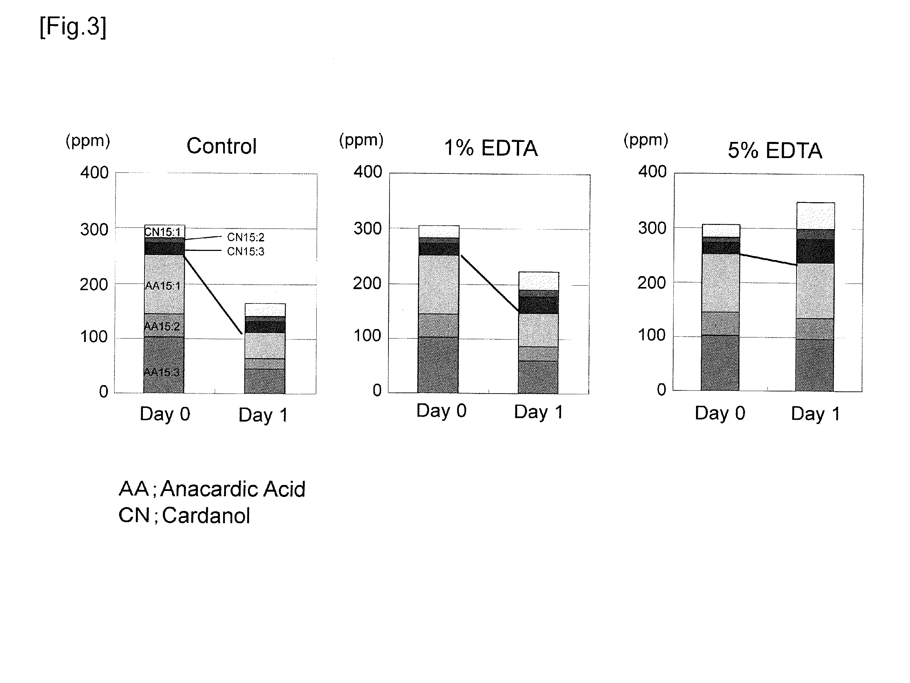

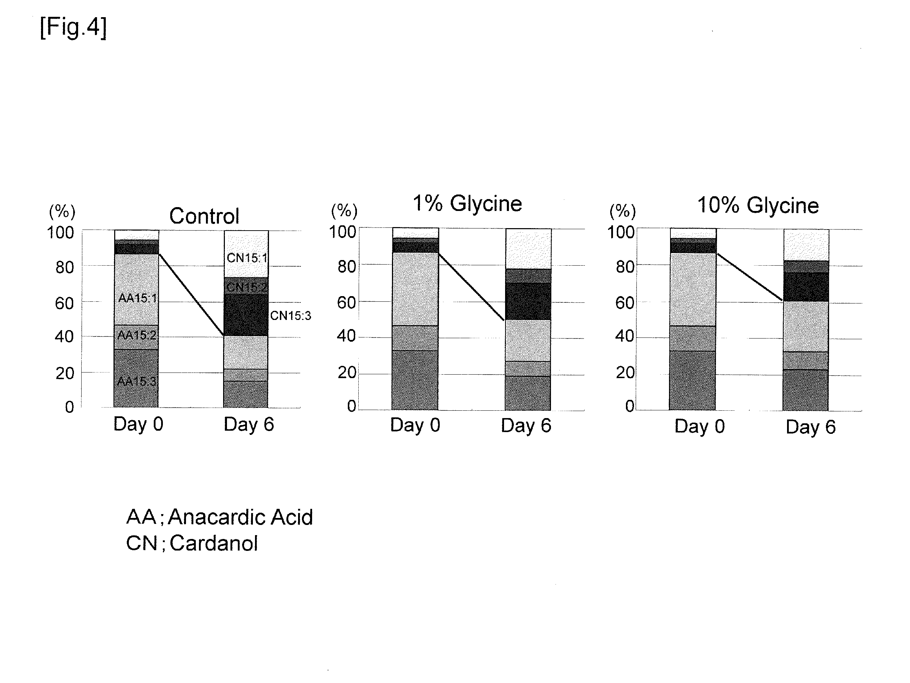

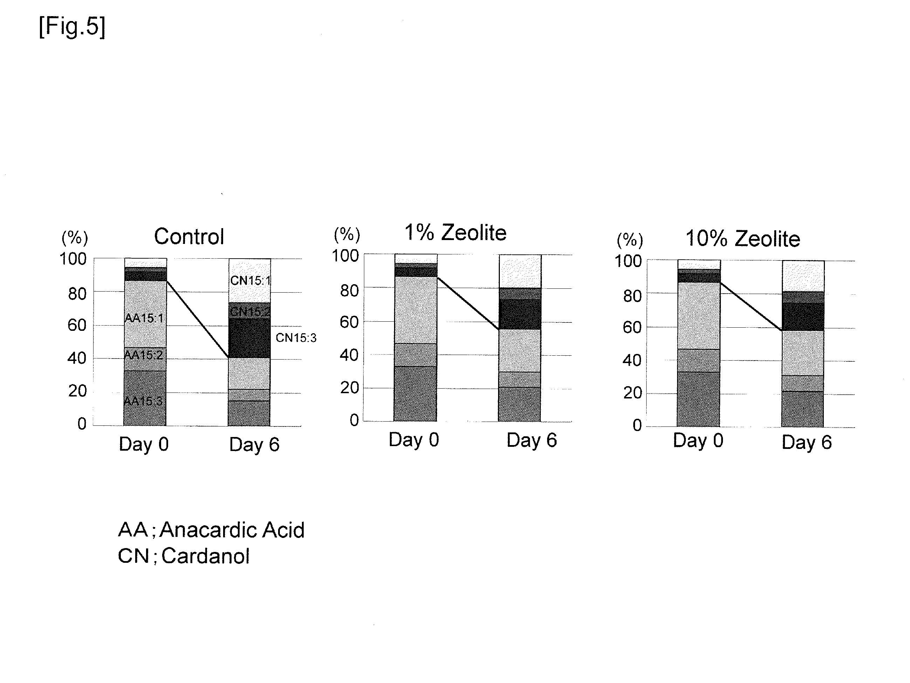

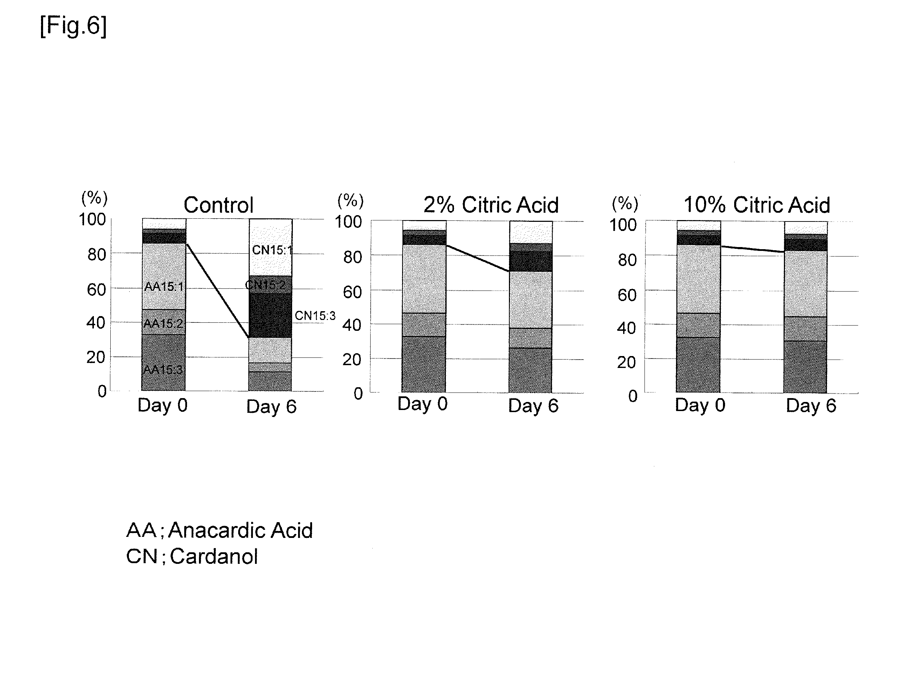

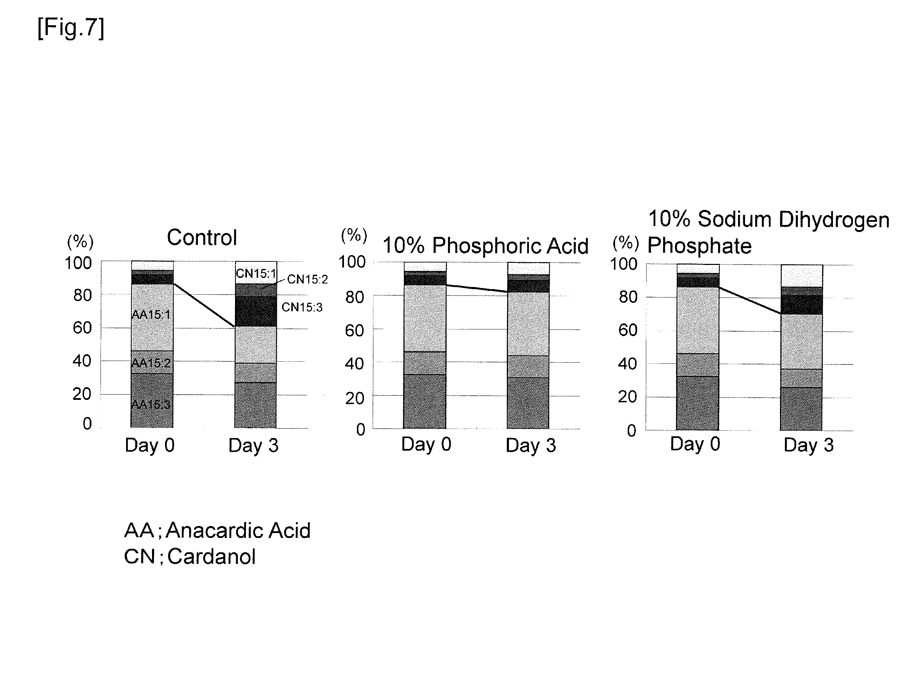

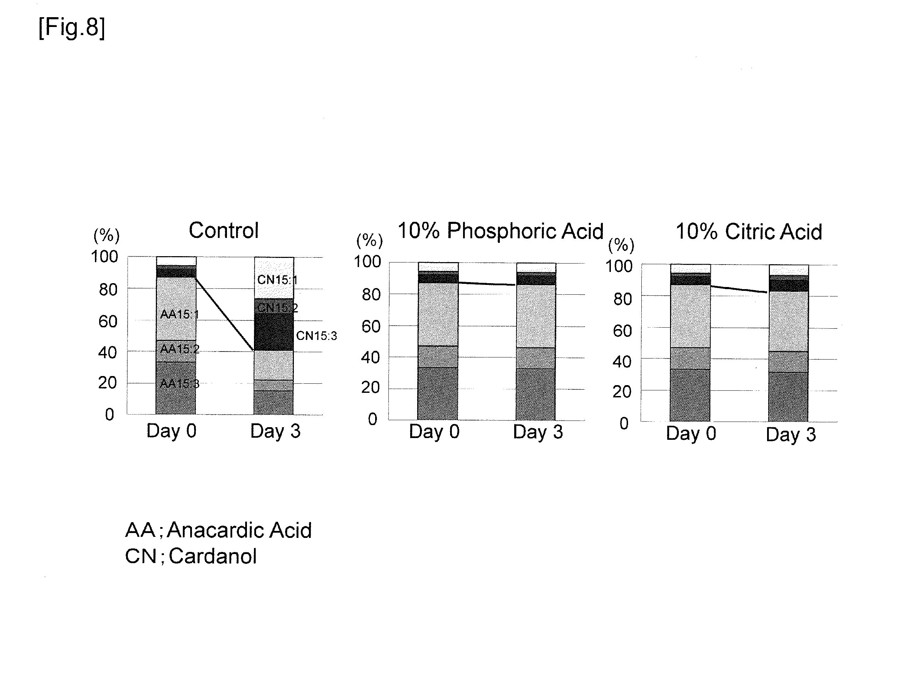

10g of the unheated cashew nut shell liquid was dispensed into each of beakers. Then 2 or 10% by weight of tartaric acid, 2 or 10% by weight of citric acid, 2 or 10% by weight of malic acid was added to the 10g of the unheated cashew nut shell liquid in the respective beaker and then stirred until homogeneous. The citric acid, the malic acid, and the tartaric acid were prepared as a 20% aqueous solution for the addition.
The beakers were placed into an 80°C incubator. Samples were taken from the beakers after six days and analyzed for composition by HPLC.
Table 1 illustrates the concentration (ppm) and the composition (%) of the anacardic acid and the cardanol before and after the incubation. There are three types of anacardic acid (AA 15:1, AA 15:2, AA 15:3) and three types of cardanol (CN 15:1, CN 15:2, CN 15:3) depending on the number of unsaturated bonds in the fatty chain which has 15 carbon atoms and which is attached to the aromatic ring. Tables 2-8 for Examples 4-10 described below also illustrate the concentration and the composition of the anacardic acid and the cardanol before and after incubation. Although Tables 1-8 correspond to FIGS. 1-8, respectively, the graphs in FIGS. 1, 2, and 4-8 indicate only the compositions and the graphs in FIG. 3 indicate only the concentrations.

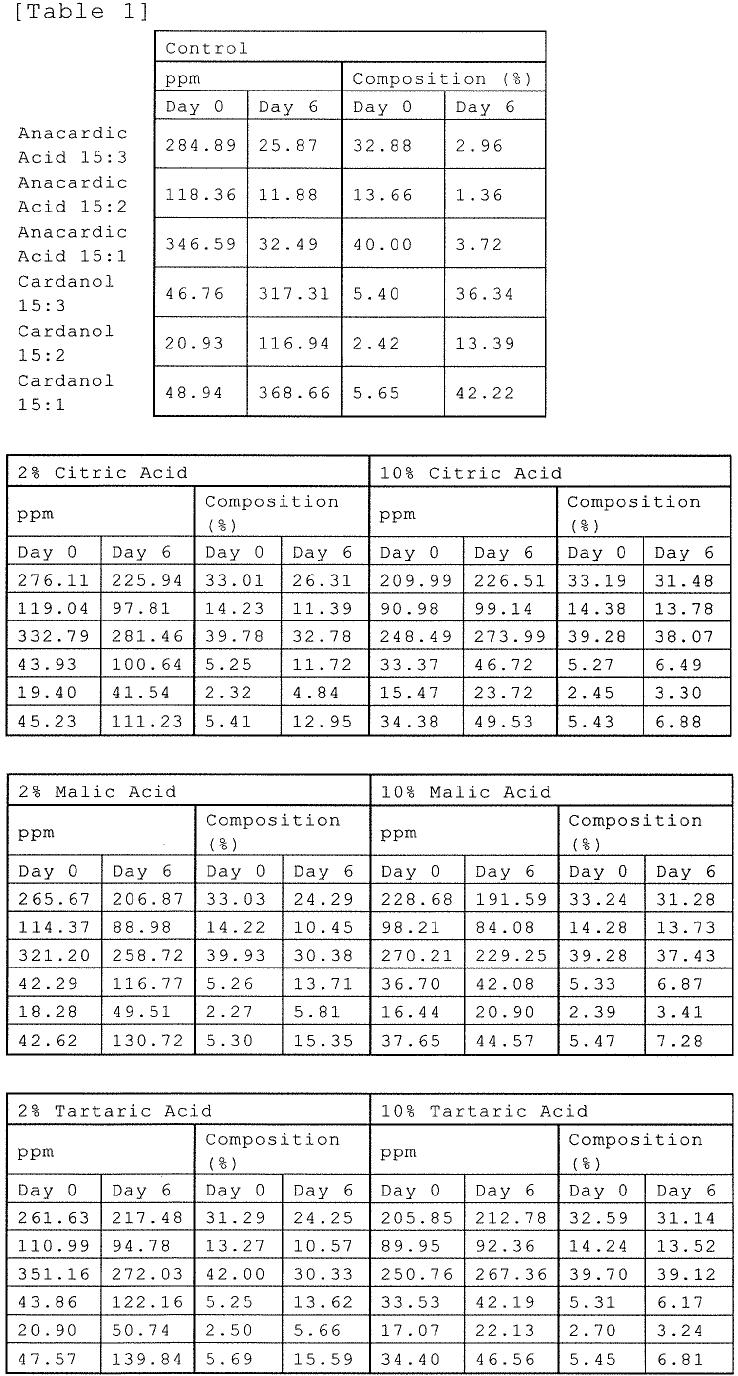

Both of the organic acids exhibited moderate inhibition of the decarboxylation (convert anacardic acid into cardanol) at the 2% by weight addition and significant inhibition of the decarboxylation at the 10% by weight addition.

### Example 4. Inhibition of Decarboxylation Reaction When Phosphoric Acid Chelating Agent and Phosphate Chelating Agent were Added

10g of the unheated cashew nut shell liquid was dispensed into each of beakers. 10% by weight of phosphoric acid or 10% by weight of sodium dihydrogen phosphate was added to the 10g of the unheated cashew nut shell liquid in the respective beaker and then stirred until homogeneous. The sodium dihydrogen phosphate was prepared as a 20% aqueous solution for the addition. A commercially-available 85% aqueous phosphoric acid solution was prepared for the addition.
The beakers were placed into an 80°C incubator. Samples were taken from the beakers after three days and analyzed for composition by HPLC.
Table 2 illustrates the concentration and the composition of the anacardic acid and the cardanol before and after the incubation.

**[Table 2]**

| | Control | | | | 10% Phosphoric Acid | | | | 10% Sodium Dihydrogen Phosphate | | | |
|---|---|---|---|---|---|---|---|---|---|---|---|---|
| | ppm | | Composition (%) | | ppm | | Composition (%) | | ppm | | Composition (%) | |
| | Day 0 | Day 3 | Day 0 | Day 3 | Day 0 | Day 3 | Day 0 | Day 3 | Day 0 | Day 3 | Day 0 | Day 3 |
| Anacardic Acid 15:3 | 1317 | 861 | 42.89 | 26.22 | 1285 | 1273 | 42.99 | 42.80 | 896 | 774 | 32.43 | 24.87 |
| Anacardic Acid 15:2 | 573 | 374 | 18.65 | 11.40 | 559 | 554 | 18.69 | 18.61 | 389 | 337 | 14.10 | 10.81 |
| Anacardic Acid 15:1 | 1001 | 664 | 32.58 | 20.21 | 978 | 968 | 32.73 | 32.55 | 1103 | 964 | 39.93 | 30.94 |
| Cardanol 15:3 | 94 | 647 | 3.05 | 19.69 | 83 | 92 | 2.78 | 3.10 | 153 | 408 | 5.55 | 13.10 |
| Cardanol 15:2 | 43 | 294 | 1.39 | 8.95 | 38 | 42 | 1.26 | 1.41 | 70 | 185 | 2.52 | 5.95 |
| Cardanol 15:1 | 45 | 445 | 1.45 | 13.54 | 46 | 46 | 1.54 | 1.53 | 151 | 446 | 5.46 | 14.32 |

As a result, addition of 10% by weight of phosphoric acid exhibited significant inhibition of the decarboxylation. Addition of 10% by weight of sodium dihydrogen phosphate exhibited moderate inhibition of the decarboxylation.

### Example 5: Inhibition of Decarboxylation Reaction When Amino Polycarboxylic Acid Chelating Agent was Added

10g of the unheated cashew nut shell liquid was dispensed into each of beakers. 1 or 5% by weight of EDTA was added to the 10g of the unheated cashew nut shell liquid in the respective beaker and then stirred until homogeneous. The EDTA was prepared as a 10% aqueous solution for the addition.
The beakers were placed into an 80°C incubator. Samples were taken from the beakers after a day and analyzed for composition by HPLC.
Table 3 illustrates the concentration and the composition of the anacardic acid and the cardanol before and after the incubation.

**[Table 3]**

| | Control | | | | 1% EDTA | | | | 5% EDTA | | | |
|---|---|---|---|---|---|---|---|---|---|---|---|---|
| | ppm | | Composition (%) | | ppm | | Composition (%) | | ppm | | Composition (%) | |
| | Day 0 | Day 1 | Day 0 | Day 1 | Day 0 | Day 1 | Day 0 | Day 1 | Day 0 | Day 1 | Day 0 | Day 1 |
| Anacardic Acid 15:3 | 101.71 | 45.30 | 33.26 | 27.41 | 101.71 | 59.67 | 33.26 | 26.75 | 101.71 | 95.61 | 33.26 | 27.51 |
| Anacardic Acid 15:2 | 42.98 | 19.14 | 14.05 | 11.58 | 42.98 | 25.79 | 14.05 | 11.56 | 42.98 | 39.59 | 14.05 | 11.39 |
| Anacardic Acid 15:1 | 107.38 | 46.12 | 35.11 | 27.91 | 107.38 | 61.63 | 35.11 | 27.63 | 107.38 | 101.34 | 35.11 | 29.16 |
| Cardanol 15:3 | 21.05 | 21.25 | 6.88 | 12.85 | 21.05 | 29.75 | 6.88 | 13.34 | 21.05 | 43.07 | 6.88 | 12.39 |
| Cardanol 15:2 | 9.45 | 9.25 | 3.09 | 5.60 | 9.45 | 12.49 | 3.09 | 5.60 | 9.45 | 18.56 | 3.09 | 5.34 |
| Cardanol 15:1 | 23.26 | 24.22 | 7.60 | 14.66 | 23.26 | 33.70 | 7.60 | 15.11 | 23.26 | 49.37 | 7.60 | 14.21 |

As a result, addition of 1% by weight of EDTA exhibited moderate inhibition of the decarboxylation, while addition of 5% by weight of EDTA exhibited significant inhibition of the decarboxylation.
In this test, the recoveries were low due to polymerization reaction. Thus the graphs in FIG. 3 indicate the concentration (ppm) rather than the composition ratio.

### Example 6: Inhibition of Decarboxylation Reaction When Neutral Amino Acid Chelating Agent was Added

10g of the unheated cashew nut shell liquid was dispensed into each of beakers. 1 or 10% by weight of glycine was added to the 10g of the unheated cashew nut shell liquid in the respective beaker and then stirred until homogeneous. The glycine was prepared as a 10% aqueous solution for the addition.
The beakers were placed into an 80°C incubator. Samples were taken from the beakers after 6 days and analyzed for composition by HPLC.
Table 4 illustrates the concentration and the composition of the anacardic acid and the cardanol before and after the incubation.

**[Table 4]**

| | Control | | | | 1% Glycine | | | | 10% Glycine | | | |
|---|---|---|---|---|---|---|---|---|---|---|---|---|
| | ppm | | Composition (%) | | ppm | | Composition (%) | | ppm | | Composition (%) | |
| | Day 0 | Day 6 | Day 0 | Day 6 | Day 0 | Day 6 | Day 0 | Day 6 | Day 0 | Day 6 | Day 0 | Day 6 |
| Anacardic Acid 15:3 | 284.89 | 46.62765 | 32.88 | 15.27 | 284.89 | 63.14 | 32.88 | 18.80 | 284.89 | 75.77 | 32.88 | 22.66 |
| Anacardic Acid 15:2 | 118.36 | 20.56413 | 13.66 | 6.74 | 118.36 | 27.85 | 13.66 | 8.29 | 118.36 | 33.42 | 13.66 | 9.99 |
| Anacardic Acid 15:1 | 346.59 | 58.20615 | 40.00 | 19.07 | 346.59 | 78.82 | 40.00 | 23.47 | 346.59 | 94.58 | 40.00 | 28.28 |
| Cardanol 15:3 | 46.76 | 71.12724 | 5.40 | 23.30 | 46.76 | 65.62 | 5.40 | 19.54 | 46.76 | 51.65 | 5.40 | 15.45 |
| Cardanol 15:2 | 20.93 | 28.87248 | 2.42 | 9.46 | 20.93 | 26.64 | 2.42 | 7.93 | 20.93 | 20.97 | 2.42 | 6.27 |
| Cardanol 15:1 | 48.94 | 79.89337 | 5.65 | 26.17 | 48.94 | 73.71 | 5.65 | 21.95 | 48.94 | 58.02 | 5.65 | 17.35 |

As a result, addition of 1 and 10% by weight of glycine exhibited subtle inhibition of the decarboxylation.

### Example 7: Inhibition of Decarboxylation Reaction When Aluminosilicate Chelating Agent was Added

10g of the unheated cashew nut shell liquid was dispensed into each of beakers. 1 or 10% by weight of zeolite was added to the 10g of the unheated cashew nut shell liquid in the respective beaker and then stirred until homogeneous.
The beakers were placed into an 80°C incubator. Samples were taken from the beakers after 6 days and analyzed for composition by HPLC.
Table 5 illustrates the concentration and the composition of the anacardic acid and the cardanol before and after the incubation.

**[Table 5]**

| | Control | | | | 1% Zeolite | | | | 10% Zeolite | | | |
|---|---|---|---|---|---|---|---|---|---|---|---|---|
| | ppm | | Composition (%) | | ppm | | Composition (%) | | ppm | | Composition (%) | |
| | Day 0 | Day 6 | Day 0 | Day 6 | Day 0 | Day 6 | Day 0 | Day 6 | Day 0 | Day 6 | Day 0 | Day 6 |
| Anacardic Acid 15:3 | 284.89 | 46.63 | 32.88 | 15.27 | 284.89 | 69.46 | 32.88 | 20.66 | 284.89 | 72.93 | 32.88 | 21.57 |
| Anacardic Acid 15:2 | 118.36 | 20.56 | 13.66 | 6.74 | 118.36 | 30.63 | 13.66 | 9.11 | 118.36 | 32.16 | 13.66 | 9.51 |
| Anacardic Acid 15:1 | 346.59 | 58.21 | 40.00 | 19.07 | 346.59 | 86.70 | 40.00 | 25.79 | 346.59 | 91.04 | 40.00 | 26.93 |
| Cardanol 15:3 | 46.76 | 71.13 | 5.40 | 23.30 | 46.76 | 59.06 | 5.40 | 17.57 | 46.76 | 56.11 | 5.40 | 16.60 |
| Cardanol 15:2 | 20.93 | 28.87 | 2.42 | 9.46 | 20.93 | 23.97 | 2.42 | 7.13 | 20.93 | 22.78 | 2.42 | 6.74 |
| Cardanol 15:1 | 48.94 | 79.89 | 5.65 | 26.17 | 48.94 | 66.34 | 5.65 | 19.73 | 48.94 | 63.02 | 5.65 | 18.64 |

As a result, addition of 1 and 10% by weight of zeolite exhibited subtle inhibition of the decarboxylation.

### Example 8: Inhibition of Decarboxylation Reaction in Unheated Cashew Nut Shell Liquid Formulation

10g of silica (Sipernat 22) was placed into each of beakers. 10g of the unheated cashew nut shell liquid or 10g of the unheated cashew nut shell liquid which included 2 or 10% by weight of citric acid was added to the respective beaker and stirred until homogeneous. The citric acid was prepared as a 50% aqueous solution for the addition.
The beakers were placed into an 80°C incubator. Samples were taken from the beakers after 6 days and analyzed for composition by HPLC.
Table 6 illustrates the concentration and the composition of the anacardic acid and the cardanol before and after the incubation.

**[Table 6]**

| | Control | | | | 2% Citric Acid | | | | 10% Citric Acid | | | |
|---|---|---|---|---|---|---|---|---|---|---|---|---|
| | ppm | | Composition (%) | | ppm | | Composition (%) | | ppm | | Composition (%) | |
| | Day 0 | Day 6 | Day 0 | Day 6 | Day 0 | Day 6 | Day 0 | Day 6 | Day 0 | Day 6 | Day 0 | Day 6 |
| Anacardic Acid 15:3 | 121.27 | 47.49 | 32.83 | 11.31 | 134.06 | 97.14 | 32.64 | 26.37 | 124.09 | 108.99 | 32.47 | 30.40 |
| Anacardic Acid 15:2 | 53.19 | 22.02 | 14.40 | 5.24 | 57.80 | 42.84 | 14.07 | 11.63 | 53.68 | 52.15 | 14.05 | 14.55 |
| Anacardic Acid 15:1 | 142.33 | 63.38 | 38.54 | 15.09 | 161.67 | 121.26 | 39.36 | 32.92 | 151.81 | 136.67 | 39.73 | 38.12 |
| Cardanol 15:3 | 20.69 | 106.69 | 5.60 | 25.41 | 22.36 | 42.34 | 5.44 | 11.49 | 20.34 | 23.49 | 5.32 | 6.55 |
| Cardanol 15:2 | 10.13 | 43.04 | 2.74 | 10.25 | 11.43 | 17.19 | 2.78 | 4.67 | 10.67 | 11.43 | 2.79 | 3.19 |
| Cardanol 15:1 | 21.72 | 137.29 | 5.88 | 32.70 | 23.41 | 47.56 | 5.70 | 12.91 | 21.54 | 25.79 | 5.64 | 7.19 |

As a result, the unheated cashew nut shell liquid formulation which included 2% by weight of citric acid exhibited moderate inhibition of the decarboxylation, while the unheated cashew nut shell liquid formulation which included 10% by weight of citric acid exhibited significant inhibition of the decarboxylation.

### Example 9: Inhibition of Decarboxylation Reaction in Unheated Cashew Nut Shell Liquid Formulation

10g of silica (Sipernat 22) was placed into each of beakers. 10g of the unheated cashew nut shell liquid or 10g of the unheated cashew nut shell liquid which included 10% by weight of phosphoric acid or 10% by weight of sodium dihydrogen phosphate was added to the respective beakers and stirred until homogeneous. The sodium dihydrogen phosphate was added as an aqueous solution.
The beakers were placed into an 80°C incubator. Samples were taken from the beakers after 3 days and analyzed for composition by HPLC.
Table 7 illustrates the concentration and the composition of the anacardic acid and the cardanol before and after the incubation.

**[Table 7]**

| | Control | | | | 10% Phosphoric Acid | | | | 10% Sodium Dihydrogen Phosphate | | | |
|---|---|---|---|---|---|---|---|---|---|---|---|---|
| | ppm | | Composition (%) | | ppm | | Composition (%) | | ppm | | Composition (%) | |
| | Day 0 | Day 3 | Day 0 | Day 3 | Day 0 | Day 3 | Day 0 | Day 3 | Day 0 | Day 3 | Day 0 | Day 3 |
| Anacardic Acid 15:3 | 451.72 | 483.68 | 32.21 | 27.10 | 451.72 | 397.52 | 32.21 | 30.69 | 451.72 | 395.86 | 32.21 | 25.93 |
| Anacardic Acid 15:2 | 196.40 | 210.30 | 14.00 | 11.78 | 196.40 | 172.83 | 14.00 | 13.34 | 196.40 | 172.11 | 14.00 | 11.28 |
| Anacardic Acid 15:1 | 563.78 | 398.27 | 40.20 | 22.32 | 563.78 | 496.13 | 40.20 | 38.30 | 563.78 | 502.26 | 40.20 | 32.90 |
| Cardanol 15:3 | 75.66 | 311.84 | 5.39 | 17.47 | 75.66 | 90.79 | 5.39 | 7.01 | 75.66 | 175.60 | 5.39 | 11.50 |
| Cardanol 15:2 | 34.39 | 141.75 | 2.45 | 7.94 | 34.39 | 41.27 | 2.45 | 3.19 | 34.39 | 79.82 | 2.45 | 5.23 |
| Cardanol 15:1 | 80.56 | 238.80 | 5.74 | 13.38 | 80.56 | 96.68 | 5.74 | 7.46 | 80.56 | 200.81 | 5.74 | 13.16 |

As a result, the unheated cashew nut shell liquid formulation which included 10% by weight of phosphoric acid exhibited significant inhibition of the decarboxylation, while the unheated cashew nut shell liquid formulation which included 10% by weight of sodium dihydrogen phosphate exhibited moderate inhibition of the decarboxylation.

### Example 10: Inhibition of Decarboxylation Reaction in Feed

1% by weight of the silica composition produced in Example 8 or 9 (10g of Sipernat 22 + 10g of the unheated cashew nut shell liquid or 10g of the unheated cashew nut shell liquid which included 10% by weight of citric acid or phosphoric acid) was added to 100g of a feed (a standard feed for young cattle: SDC No. 2 from Nippon Formula Feed Manufacturing Co. Ltd. ) to produce a feed which included the unheated cashew nut shell liquid. The citric acid was added as an aqueous solution.
The beakers were placed into an 80°C incubator. Samples were taken from the beakers after 3 days and analyzed for composition by HPLC.
For HPLC analysis, soluble fractions of the test feed was extracted with ethyl acetate and then filtered and dried to concentrate it to 5 mg/ml.
Table 8 illustrates the concentration and the composition of the anacardic acid and the cardanol before and after the incubation.

**[Table 8]**

| | Control | | | | 10% Phosphoric Acid | | | | 10% Citric Acid | | | |
|---|---|---|---|---|---|---|---|---|---|---|---|---|
| | ppm | | Composition (%) | | ppm | | Composition (%) | | ppm | | Composition (%) | |
| | Day 0 | Day 3 | Day 0 | Day 3 | Day 0 | Day 3 | Day 0 | Day 3 | Day 0 | Day 3 | Day 0 | Day 3 |
| Anacardic Acid 15:3 | 299.13 | 46.63 | 33.11 | 15.27 | 299.13 | 284.18 | 33.11 | 32.67 | 299.13 | 254.26 | 33.11 | 31.68 |
| Anacardic Acid 15:2 | 124.28 | 20.56 | 13.76 | 6.74 | 124.28 | 118.06 | 13.76 | 13.57 | 124.28 | 105.64 | 13.76 | 13.16 |
| Anacardic Acid 15:1 | 363.92 | 58.21 | 40.28 | 19.07 | 363.92 | 345.72 | 40.28 | 39.75 | 363.92 | 309.33 | 40.28 | 38.54 |
| Cardanol 15:3 | 46.52 | 71.13 | 5.15 | 23.30 | 46.52 | 48.85 | 5.15 | 5.62 | 46.52 | 53.50 | 5.15 | 6.67 |
| Cardanol 15:2 | 20.83 | 28.87 | 2.31 | 9.46 | 20.83 | 21.87 | 2.31 | 2.51 | 20.83 | 23.95 | 2.31 | 2.98 |
| Cardanol 15:1 | 48.69 | 79.89 | 5.39 | 26.17 | 48.69 | 51.13 | 5.39 | 5.88 | 48.69 | 56.00 | 5.39 | 6.98 |

It has found that use of the unheated cashew nut shell liquid which included 10% by weight of citric acid or phosphoric acid allowed the shell liquid to be stable in the feed without progress of the decarboxylation.

Although, in the above test, the feed which included the cashew nut shell liquid was placed into an 80°C incubator to evaluate decarboxylation of the anacardic acid for reduced testing time, it will be appreciated that unheated cashew nut shell liquid can cause decarboxylation of the anacardic acid at room temperature, and thus addition of a chelating agent allows inhibition of decarboxylation of the anacardic acid in unheated cashew nut shell liquid.

### INDUSTRIAL APPLICABILITY

Addition of a chelating agent to unheated cashew nut shell liquid allows inhibition of foaming due to decarboxylation. Addition of an inorganic carrier to unheated cashew nut shell liquid in addition to the chelating agent allows inhibition of solidification of the unheated cashew nut shell liquid at about 20°C. These facilitate industrial use of unheated cashew nut shell liquid.

## Claims

1. A composition comprising a chelating agent and unheated cashew nut shell liquid.

2. The composition according to claim 1, wherein the chelating agent is an organic acid chelating agent, an organic acid salt chelating agent, a phosphoric acid chelating agent, a phosphate chelating agent, an amino polycarboxylic acid chelating agent, an amino polycarboxylate chelating agent, a phosphonic acid chelating agent, a phosphonate chelating agent, a neutral amino acid chelating agent, an aluminosilicate chelating agent, or a polymer chelating agent.

3. The composition according to claim 2, wherein the organic acid chelating agent and the organic acid salt chelating agent are citric acid or a salt thereof, malic acid or a salt thereof, tartaric acid or a salt thereof, succinic acid or a salt thereof, propionic acid or a salt thereof, gluconic acid or a salt thereof, oxalic acid or a salt thereof, or glycolic acid or a salt thereof.

4. The composition according to claim 3, wherein the chelating agent is citric acid, malic acid, or tartaric acid.

5. The composition according to claim 2, wherein the phosphoric acid chelating agent and the phosphate chelating agent are orthophosphoric acid or a salt thereof, pyrophosphoric acid or a salt thereof, tripolyphosphoric acid or a salt thereof, tetrapolyphosphoric acid or a salt thereof, hexametaphosphoric acid or a salt thereof, or phytic acid or a salt thereof.

6. The composition according to claim 5, wherein the chelating agent is orthophosphoric acid or a salt thereof.

7. The composition according to claim 2, wherein the amino polycarboxylic acid chelating agent and the amino polycarboxylate chelating agent are ethylenediamine tetraacetic acid or a salt thereof, ethylenediamine diacetic acid or a salt thereof, hydroxyethyl ethylenediamine tetraacetic acid or a salt thereof, diethylenetriamine pentaacetic acid or a salt thereof, nitrilotriacetic acid or a salt thereof, triethylenetetraamine hexaacetic acid or a salt thereof, dicarboxymethyl glutamine hexaacetic acid or a salt thereof, dicarboxymethyl glutamic acid tetrasodium salt, or dihydroxymethyl glycine.

8. The composition according to claim 7, wherein the chelating agent is ethylenediamine tetraacetic acid.

9. The composition according to claim 2, wherein the neutral amino acid chelating agent is glycine, alanine, leucine, cysteine, methionine, asparagine, or glutamine.

10. The composition according to claim 9, wherein the chelating agent is glycine.

11. The composition according to claim 2, wherein the aluminosilicate chelating agent is zeolite.

12. The composition according to claim 2, wherein the polymer chelating agent is polyacrylic acid, polymaleic acid, or a copolymer of acrylic acid and maleic acid.

13. The composition according to any one of claims 1-12, wherein an amount of the chelating agent is equal to or greater than 0.01% by weight based on the weight of the cashew nut shell liquid.

14. An unheated cashew nut shell liquid formulation comprising the composition according to any one of claims 1-13 and an inorganic carrier.

15. The unheated cashew nut shell liquid formulation according to claim 14, wherein the inorganic carrier is silica.

16. A feed containing unheated cashew nut shell liquid, comprising the composition according to any one of claims 1-13 and/or the unheated cashew nut shell liquid formulation according to claim 14 or 15.

17. A method for inhibiting decarboxylation reaction of the anacardic acid in unheated cashew nut shell liquid, comprising adding a chelating agent to the unheated cashew nut shell liquid.

18. A method for producing the unheated cashew nut shell liquid formulation according to claim 14 or 15, comprising mixing a chelating agent with an inorganic carrier and then mixing the mixture of the chelating agent and the inorganic carrier with unheated cashew nut shell liquid.

19. A method for inhibiting decarboxylation reaction of anacardic acid, comprising adding a chelating agent to the anacardic acid.
